# EUROPÄISCHE PATENTSCHRIFT

(11) **EP 0 945 430 B1**
(45) Veröffentlichungstag und Bekanntmachung des Hinweises auf die Patenterteilung: **28.05.2003**
(21) Anmeldenummer: 99104842.2
(22) Anmeldetag: 11.03.1999
(51) Int. Cl.: C07C 235/80, C07C 231/04

(54) **N-phenylacetoacetamide und Verfahren zu ihrer Herstellung**
N-phenylacetoacetamides and process for their preparation
Acéto-acétamides N-phéniliques et procédé pour leur préparation

(30) Priorität: 25.03.1998 CH 70898
(43) Veröffentlichungstag der Anmeldung: 29.09.1999
(73) Patentinhaber: Lonza AG, 4052 Basel (CH)
(72) Erfinder: Balmer, Bernard, Dr., 3930 Visp (CH); Hafkesbrink, Sven, Dr., 3912 Termen (CH); Lauwiner, Max, Dr., 3900 Brig (CH)

(56) Entgegenhaltungen:
- EP-A- 0 648 738
- DE-A- 2 519 036
- US-A- 2 152 132

## Beschreibung

Die Erfindung betrifft ein neues Verfahren zur Herstellung von Acetessigsäurearylamiden der allgemeinen Formel 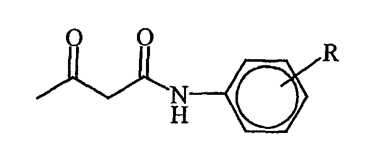 worin R einen oder mehrere Substituenten aus der Reihe Alkyl, Alkoxy oder Halogen bedeutet.

Acetessigsäurearylamide sind wichtige Ausgangsprodukte zur Herstellung von Farbpigmenten, dienen aber auch zur Herstellung von Agrowirkstoffen (Ullmann's Encyclopedia of Industrial Chemistry, 5. Auflage, Vol. 15, S. 71).

Die Herstellung von Acetessigsäurearylamiden ist seit langem bekannt und basiert auf der Umsetzung von Diketen mit entsprechenden aromatischen Aminen in verschiedenen organischen und wässrigen Lösungsmitteln und Lösungsmittelgemischen (Ullmann's Encyclopedia of Industrial Chemistry, 5. Auflage, Vol. 15, S. 71).
In der Regel wird diskontinuierlich in Wasser oder wässrigen Lösungen gearbeitet. Dementsprechend offenbart die DE-A 25 19 036 die Herstellung von verschiedenen Acetessigsäurearylamiden durch simultane Zudosierung von Diketen und dem entsprechenden aromatischen Amin in Gegenwart von Wasser oder wässrigen Lösungen. Das resultierende Acetessigsäurearylamid wird im Reaktionsmedium gekühlt und auskristallisieren gelassen. Nach Zentrifugation und Trocknung werden die Acetessigsäurearylamide in guter Ausbeute und hoher Reinheit erhalten.

Aus der EP-A 0 648 738 ist ausserdem ein kontinuierliches Verfahren zur Herstellung von Acetessigsäurearylamiden bekannt. Darin wird Diketen kontinuierlich mit dem aromatischen Amin in einem Gemisch Wasser/Alkohol unter möglichst langer Verweilzeit des Reaktionsgemisches im Reaktor hergestellt. Das resultierende Acetessigsäurearylamid wird durch Kristallisation des aus dem Reaktor ausgeschleusten Produktstroms gewonnen.

Das gemäss dem klassischen Verfahren hergestellte Acetessigsäurearylamid erfüllt alle Anforderungen bezüglich Qualität und Reinheit. Es stellte sich aber heraus, dass für eine Weiterverarbeitung, z.B. für die Pigmentherstellung, die in der Regel das Auflösen der Acetessigsäurearylamide in wässrigen Alkalien beinhaltet, die feinkristalline Pulverform eher einen Nachteil bedeutet. So ist neben der unangenehmen Staubbildung die langsame Lösungsgeschwindigkeit in wässrigen Alkalien eher unbefriedigend.

Die Aufgabe der Erfindung bestand folglich darin, die Acetessigsäurearylamide in einer Form herzustellen, die die genannten Nachteile nicht beinhaltet. Ausserdem war es Aufgabe der Erfindung, ein wirtschaftliches Verfahren zur Verfügung zu stellen, das eine Acetessigsäurearylamid-Herstellung mit geringerem Kostenaufwand erlaubt.

Die Aufgabe konnte erfindungsgemäss gelöst werden mit den Acetessigsäurearylamiden in der Form und mit den Eigenschaften gemäss Patentanspruch 1.

Die Acetessigsäurearylamide sind definiert durch die allgemeine Formel 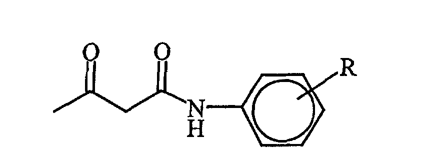 worin R Wasserstoff oder einen oder mehrere Substituenten aus der Reihe Alkyl, Alkoxy oder Halogen bedeutet, liegen als erstarrte Schmelze in einer industriell anwendbaren und handhabbaren Form vor und weisen einen Wassergehalt zwischen 5 Gew.% und 15 Gew.% auf.

Zweckmässig wird unter einer Alkylgruppe eine C₁₋₄-Alkylgruppe, namentlich Methyl, Ethyl, n-Propyl, Isopropyl, n-Butyl, Isobutyl oder tert-Butyl verstanden. Bevorzugtes Alkyl ist Methyl. Der Alkylgruppe entsprechende Bedeutung kommt der Alkoxygruppe zu. Bevorzugt ist hier Methoxy. Halogen steht für Fluor, Chlor, Brom oder Iod, bevorzugt für Chlor. Besonders bevorzugte Arylamide sind die vom Anilin, o-Anisidin, o-Toluidin, m-Xylidin, o-Chloranilin, 2,4-Dimethoxyanilin, 4-Isopropylanilin, 4-Ethoxyanilin, 2,5-Dimethoxyanilin oder 4-Chlor-2,5-dimethoxyanilin abgeleiteten.
Der Begriff industriell anwendbare und handhabbare Form umfasst solche Anwendungsformen wie sie mittels in der Fachwelt üblicher Methoden aus einer erstarrten Schmelze gewonnen werden können. Beispielhaft und nicht abschliessend seien aufgeführt: Pastillen, Schuppen, Tabletten oder Prillen. Die Dimension und Grösse der genannten Anwendungsformen ist abhängig von den genannten Pastillier-, Schupp-, Tablettier- oder Prillierverfahren und kann in einem weiten Bereich variieren. Die erfindungsgemässen Acetessigsäurearylamide weisen jedenfalls eine im Vergleich zu einem Acetessigsäurearylamid gemäss dem Stand der Technik verbesserte Rieselfähigkeit auf.

Das Schüttgewicht der erfindungsgemässen Acetessigsäurearylamide liegt zweckmässig im Bereich von 0,3 kg/l bis 0,8 kg/l, bevorzugt im Bereich von 0,5 kg/l bis 0,7 kg/l.

Der genannte Begriff Wassergehalt kann auch einen Gehalt einer beliebigen Mischung von Wasser mit einem gegebenenfalls aus der Reaktion stammenden zusätzlichen Lösungsvermittler, wie z. B. einer aliphatischen Carbonsäure wie Essigsäure, einem Keton wie Aceton oder Ethylmethylketon, einem C₁₋₄-Alkohol oder einem Glykol wie Ethylenglykol, bedeuten.

Die erfindungsgemässen Acetessigsäurearylamide weisen einen, im Vergleich zum getrockneten Produkt, erniedrigten charakteristischen Schmelzpunkt auf.

Die erfindungsgemässen Acetessigsäurearylamide lösen sich in einer 0,5 N Natronlauge bei 20 °C in etwa der Hälfte bis zu einen Drittel des Zeitbedarfs gegenüber einem nach dem Stand der Technik hergestellten getrockneten Acetessigsäurearylamid auf.

Bevorzugte Acetessigsäurearylamide mit ihren charakteristischen Eigenschaften sind:
Acetessigsäureanilid, Wassergehalt: zweckmässig 5 Gew.% bis 15 Gew.%, bevorzugt 9 Gew.% bis 11 Gew.%; Schmelzpunkt: zweckmässig 50 °C bis 70 °C, bevorzugt 57 °C bis 62 °C; Löslichkeit in 500 ml 0,5 N NaOH: 60 g Pastillen mit Durchmesser 0,6 cm 10 bis 20 Minuten.
Acetessigsäure-o-anisidid, Wassergehalt: zweckmässig bis zu 12 Gew.%, bevorzugt 6 Gew.% bis 9 Gew.%; Schmelzpunkt: zweckmässig 60 °C bis 80 °C, bevorzugt 72 °C bis 75 °C; Löslichkeit in 500 ml 0,5 N NaOH: 60 g Pastillen mit Durchmesser 0,6 cm 10 bis 20 Minuten.
Acetessigsäure-o-toluidid, Wassergehalt: zweckmässig 5 Gew.% bis 15 Gew.%, bevorzugt 8 Gew.% bis 12 Gew.%; Schmelzpunkt: zweckmässig 80 °C bis 100 °C, bevorzugt 82 °C bis 94 °C; Löslichkeit in 700 ml 0,5N NaOH: 60 g Pastillen mit Durchmesser 0,6 cm 10 bis 20 Minuten.
Acetessigsäure-m-xylidid, Wassergehalt: zweckmässig bis zu 12 Gew.%, bevorzugt bis zu 8 Gew.%; Schmelzpunkt: zweckmässig 65 °C bis 85 °C, bevorzugt 68 °C bis 73 °C; Löslichkeit in 700 ml 0,5N NaOH: 60 g Pastillen mit Durchmesser 0,6 cm 10 bis 20 Minuten.
Acetessigsäure-o-chloranilid, Wassergehalt: zweckmässig bis zu 12 Gew.%, bevorzugt 5 Gew.% bis 8 Gew.%; Schmelzpunkt: zweckmässig 75 °C bis 95 °C, bevorzugt 89 °C bis 93 °C; Löslichkeit in 500 ml 0,5N NaOH: 60 g Pastillen mit Durchmesser 0,6 cm 10 bis 20 Minuten.

Die Herstellung der erfindungsgemässen Acetessigsäurearylamide erfolgt zunächst auf bekannte Weise durch Umsetzung von Diketen mit dem entsprechenden aromatischen Amin in Gegenwart von Wasser oder wässrigen Lösungen von Wasser mit einem geeigneten Lösungsvermittler wie z. B. Essigsäure. Gemäss der vorliegenden Erfindung wird die Reaktion so geführt, dass das resultierende Acetessigsäurearylamid als Schmelze anfällt, die Schmelze nach beendeter Reaktion bei Reaktionstemperatur vom Reaktionsmedium getrennt und nach Kühlung in die industriell anwendbare und handhabbare Form überführt wird.
Die Herstellung kann diskontinuierlich, z. B. in einem klassischen Rührwerk, oder kontinuierlich, z. B. in einem Rohrreaktor, erfolgen. Bevorzugt erfolgt die Umsetzung kontinuierlich in einem Rohrreaktor.
Besonders bevorzugt werden Diketen und das entsprechende aromatische Amin simultan zu im Kreis geführter Mutterlauge in einen Rohrreaktor so dosiert, dass die Reaktionstemperatur bei 50 °C bis 100 °C gehalten wird. Die Mutterlauge setzt sich dabei zweckmässig aus mindestens 80 Gew.% Wasser zusammen. Nach der Reaktion wird gemäss dem bevorzugten Verfahren bei 50 °C bis 100 °C die wässrige Mutterlauge abgetrennt und wieder dem Rohrreaktor zugeführt. Die Acetessigsäurearylamidschmelze mit der Temperatur von 50 °C bis 100 °C wird darauf durch Kühlen erstarrt und in die industriell anwendbare und handhabbare Form überführt. Dies kann z.B. mit in der Fachwelt üblichen Trommelkühlern, die mit einer Schuppvorrichtung versehen sind, mit Bandkühlern, die mit einer Schuppvorrichtung ausgerüstet sind, oder mit Bandpastillierern erfolgen.

### Beispiele

### Beispiel 1

### Herstellung von Acetessigsäureanilid (Vergleichsbeispiel gemäss DE-A 25 19 036)

In einem 1,5 l Labor-Rührwerk wurde Wasser vorgelegt und auf eine Temperatur von 20 °C eingestellt. Dann wurde das Rührwerk mit Stickstoff inertisiert. Mit zwei Pumpen wurden 133 g Anilin und 124 g Diketen während 10 Minuten zudosiert. Die Anilin-Zugabe wurde um ca. 30 s retardiert. Am Ende der Dosierung stieg die Reaktionstemperatur auf 65-70 °C. Man liess 30 Minuten nachreagieren, worauf die Reaktionstemperatur auf 60 °C sank. Mittels Vakuumkühlung (450-120 mbar) wurde auf 40 °C gekühlt, wobei das flüssige Acetessigsäureanilid zu kristallisieren begann, wodurch die Temperatur kurzfristig auf etwa 58 °C stieg. Darauf kühlte man auf 15 °C. Bei 15 °C wurde das feste Produkt abzentrifugiert, mit 50 ml Wasser gewaschen und bei 50 °C unter Vakuum während 12 h getrocknet. Auf diese Weise liess sich Acetessigsäureanilid mit einem Gehalt von >99,5% (bei einem Restfeuchtegehalt von <0,1%) herstellen. Die Ausbeute bezüglich Anilin betrug 97,1% respektive bezüglich Diketen 92,0%. Man erhält ein kristallines Produkt mit einer mittleren Korngrösse von 450-500 µm.
50 g des so erhaltenen Acetessigsäureanilid (trocken, 99,5%, Wassergehalt <0,1%) lösen sich bei 20 °C in 500 ml einer 0,5 M wässrigen Natronlauge in 25 Minuten vollständig.

### Beispiel 2

### Herstellung der erstarrten Schmelze von Acetessigsäureanilid (Rohrreaktor)

In einem Rohrreaktor (Länge 80 cm, Durchmesser 10 cm) wurden 1300 ml Wasser bei 62 °C im Kreis geführt und simultan 0,200 g/s Anilin und 0,190 g/s Diketen (5 Gew.% Überschuss) zudosiert. Nachdem die Reaktionsmischung den Rohrreaktor durchlaufen hatte, wurde sie in ein auf 62 °C thermostatisiertes Abscheidegefäss gelassen. Die Schmelze wurde dann von der Wasserphase abgetrennt. Die Wasserphase wurde über eine Pumpe mit 2250 ml/min Umwälzgeschwindigkeit wiederum dem Rohrreaktor zugeführt. Die abgetrennte Schmelze wurde sofort erstarrt und gemäss den Beispielen 4 bis 6 weiterverarbeitet. Man erhielt eine Schmelze mit einem Wassergehalt von 11 Gew.% und einem Schmelzpunkt von 62 °C.

Zermörsern der erstarrten Schmelze und Trocknen im Vakuum während 12 h bei 50 °C führte zu einem kristallinen Acetessigsäureanilid mit einem Gehalt von >99,5 Gew.% (bei einem Restfeuchtegehalt von <0,1%). Die Ausbeute bezüglich Anilin betrug 99,1%, respektive bezüglich Diketen je nach Diketenüberschuss zwischen 94,0 und 98,7%.

### Beispiel 3

### Herstellung der erstarrten Schmelze von Acetessigsäureanilid (Laborrührwerk)

In einem 1,5 l Labor-Rührwerk wurde Wasser vorgelegt und auf eine Temperatur von 20 °C eingestellt. Dann wurde das Rührwerk mit Stickstoff inertisiert. Mit zwei Pumpen wurden 133 g Anilin und 124 g Diketen während 10 Minuten zudosiert. Die Anilin-Zugabe wurde um ca. 30 s retardiert. Am Ende der Dosierung stieg die Reaktionstemperatur auf 65-70 °C. Man liess darauf während 30 Minuten nachreagieren, worauf die Reaktionstemperatur auf 60 °C sank. Die Schmelze wurde abgelassen und ausgekühlt. Man erhielt eine Schmelze mit einem Wassergehalt von 10 Gew.%. und einem Schmelzpunkt von 61,5 °C.

Zermörsern der erstarrten Schmelze und Trocknen im Vakuum während 12 h bei 50 °C führte zu einem kristallinen Acetessigsäureanilid mit einem Gehalt von >99,5 Gew.% (bei einem Restfeuchtegehalt von <0,1%). Die Ausbeuten bezüglich Anilin betrugen 80,5%, respektive bezüglich Diketen 76%.

### Beispiel 4

### Schuppen der erstarrten Schmelze von Acetessigsäureanilid mit einem Trommelkühler

Die gemäss Beispiel 3 hergestellte Schmelze wurde bei einer Temperatur von 60 °C kontinuierlich auf eine rotierende, mit externem Kühlkreislauf auf 0 °C gekühlte, Metallwalze mit glatter Oberfläche, einem Durchmesser von 30 cm und einer Umdrehungszahl von 10 U/min aufgetropft, dabei sofort erstarrt und nach einer Kühlstrecke von 30 cm mit einem Messer von der Walze abgeschuppt. Man erhielt weisse Schuppen mit einer mittleren Dicke von 0,08 mm und einem mittleren Durchmesser von 0,5 mm bis 500 mm. 55,6 g des so erhaltenen Acetessigsäureanilids (feucht, Gehalt an Acetessigsäureanilid 89,5%, Wassergehalt 10%) lösen sich bei 20 °C in 500 ml einer 0,5 M wässrigen Natronlauge in etwa 15 Minuten vollständig auf.

### Beispiel 5

### Schuppen der erstarrten Schmelze von Acetessigsäureanilid mit einem Bandkühler

Die gemäss Beispiel 3 hergestellte Schmelze wurde bei einer Temperatur von 60 °C kontinuierlich auf ein rotierendes, mit externem Kühlkreislauf auf 0 °C gekühltes, Metallband mit glatter Oberfläche, einer Breite von 30 cm und einer Umlaufgeschwindigkeit von 10 m/min aufgetropft, dabei sofort erstarrt und nach einer Kühlstrecke von 4 m mit einem Messer vom Bandkühler abgeschuppt. Man erhielt weisse Schuppen mit einer mittleren Dicke von 0,08 cm und einem mittleren Durchmesser von 0,5 mm bis 500 mm. 55,6 g des so erhaltenen Acetessigsäureanilids (feucht, Gehalt an Acetessigsäureanilid 89,5%, Wassergehalt 10%) lösen sich bei 20 °C in 500 ml einer 0,5 M wässrigen Natronlauge in etwa 17 Minuten vollständig auf.

### Beispiel 6

### Pastillen der erstarrten Schmelze von Acetessigsäureanilid mit einem Bandpastillierer

Die gemäss Beispiel 3 hergestellte Schmelze wurde bei einer Temperatur von 60 °C mittels einer für die Herstellung von Pastillen geeigneten Vorrichtung kontinuierlich auf ein rotierendes, mit externem Kühlkreislauf auf 0 °C gekühltes, Metallband mit glatter Oberfläche, einer Breite von 30 cm und einer Umlaufgeschwindigkeit von 10 m/min aufgetropft, dabei sofort erstarrt und nach einer Kühlstrecke von 4 m vom Band genommen. Man erhielt weisse Pastillen mit einer mittleren Dicke von 0,05 cm und einem mittleren Durchmesser von 0,6 cm. 55,6 g des so erhaltenen Acetessigsäureanilids (feucht, Gehalt an Acetessigsäureanilid 89,5%, Wassergehalt 10%) lösen sich bei 20 °C in 500 ml einer 0,5 M wässrigen Natronlauge in etwa 17 Minuten vollständig auf.

### Beispiel 7

### Herstellung der erstarrten Schmelze von Acetessigsäure-o-anisidid

Analog Beispiel 2 wurde durch entsprechende Umsetzung von Diketen und o-Anisidin eine Schmelze mit einem Wassergehalt von 7 Gew.% und einem Schmelzpunkt von 74 °C erhalten, welche gemäss Beispiel 6 zu Pastillen verarbeitet wurde.
60 g des so erhaltenen Acetessigsäure-o-anisidids (feucht, Gehalt an Acetessigsäure-o-anisidid 91,8%, Wassergehalt 8%) lösen sich bei 20 °C in 500 ml einer 0,5 M wässrigen Natronlauge in etwa 20 Minuten.

### Beispiel 8

### Herstellung der erstarrten Schmelze von Acetessigsäure-o-toluidid

Analog Beispiel 2 wurde durch entsprechende Umsetzung von Diketen und o-Toluidin eine Schmelze mit einem Wassergehalt von 10 Gew.% und einem Schmelzpunkt von 85 °C erhalten, welche gemäss Beispiel 6 zu Pastillen verarbeitet wurde.
60 g des so erhaltenen Acetessigsäure-o-toluidids (feucht, Gehalt an Acetessigsäure-o-toluidid 89,6%, Wassergehalt 10%) lösen sich bei 20 °C in 700 ml einer 0,5 M wässrigen Natronlauge in etwa 19 Minuten.

### Beispiel 9

### Herstellung der erstarrten Schmelze von Acetessigsäure-m-xylidid

Analog Beispiel 2 wurde durch entsprechende Umsetzung von Diketen und m-Xylidin eine Schmelze mit einem Wassergehalt von 7 Gew.% und einem Schmelzpunkt von 74 °C erhalten, welche gemäss Beispiel 6 zu Pastillen verarbeitet wurde.
60 g des so erhaltenen Acetessigsäure-m-xylidids (feucht, Gehalt an Acetessigsäure-m-xylidid 92,5%, Wassergehalt 7%) lösen sich bei 20 °C in 700 ml einer 0,5 M wässrigen Natronlauge in etwa 18 Minuten.

### Beispiel 10

### Herstellung der erstarrten Schmelze von Acetessigsäure-o-chloranilid

Analog Beispiel 2 wurde durch entsprechende Umsetzung von Diketen und o-Chloranilin eine Schmelze mit einem Wassergehalt von 7 Gew.% und einem Schmelzpunkt von 90 °C erhalten, welche gemäss Beispiel 6 zu Pastillen verarbeitet wurde.
60 g des so erhaltenen Acetessigsäure-o-chloranilids (feucht, Gehalt an Acetessigsäure-o-chloranilid 92,7%, Wassergehalt 7%) lösen sich bei 20 °C in 500 ml einer 0,5 M wässrigen Natronlauge in etwa 18 Minuten.

## Patentansprüche

1. Acetessigsäurearylamide definiert durch die allgemeine Formel 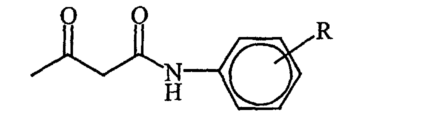 worin R Wasserstoff oder eine oder mehrere Substituenten aus der Reihe Alkyl, Alkoxy oder Halogen bedeutet, als erstarrte Schmelze in einer industriell anwendbaren und handhabbaren Form und mit einem Wassergehalt zwischen 5 Gew.% und 15 Gew.%.

2. Acetessigsäurearylamide gemäss Patentanspruch 1 in Form von Schuppen, Prillen, Pastillen oder Tabletten.

3. Acetessigsäurearylamide gemäss Patentanspruch 1 oder 2 mit einem Schüttgewicht zwischen 0,3 kg/l und 0,8 kg/l.

4. Acetessigsäureanilid als Acetessigsäurearylamid gemäss einem der Patentansprüche 1 bis 3 mit einem Schmelzpunkt von 50 °C bis 70 °C.

5. Acetessigsäure-o-anisidid als Acetessigsäurearylamid gemäss einem der Patentansprüche 1 bis 3 mit einem Wassergehalt von bis zu 12 Gew.% und einem Schmelzpunkt von 50 °C bis 80 °C.

6. Acetessigsäure-o-toluidid als Acetessigsäurearylamid gemäss einem der Patentansprüche 1 bis 3 mit einem Schmelzpunkt von 80 °C bis 100 °C.

7. Acetessigsäure-m-xylidid als Acetessigsäurearylamid gemäss einem der Patentansprüche 1 bis 3 mit einem Wassergehalt von bis zu 12 Gew.% und einem Schmelzpunkt von 65 °C bis 85 °C.

8. Acetessigsäure-o-chloranilid als Acetessigsäurearylamid gemäss einem der Patentansprüche 1 bis 3 mit einem Wassergehalt von bis zu 12 Gew.% und einem Schmelzpunkt von 75 °C bis 95 °C.

9. Acetessigsäurearylamide gemäss Patentansprüchen 1 bis 8, erhältlich durch Umsetzung von Diketen und dem entsprechenden aromatischen Amin in Gegenwart einer wässrigen Mutterlauge, die sich zu mindestens 80 Gew.% aus Wasser zusammensetzt, wobei Diketen und aromatisches Amin simultan zu im Kreis geführter Mutterlauge in einen Rohrreaktor so dosiert werden, dass die Reaktionstemperatur bei 50 °C bis 100 °C gehalten wird, die Mutterlauge nach der Reaktion bei der genannten Temperatur von 50 °C bis 100 °C abgetrennt und wieder dem Rohrreaktor zugeführt wird, und die erhaltene Acetessigsäurearylamidschmelze mit einer Temperatur von 50 °C bis 100 °C darauf gekühlt und in die industriell anwendbare und handhabbare Form gebracht wird.

10. Verfahren zur Herstellung von Acetessigsäurearylamiden durch die Umsetzung von Diketen und dem entsprechenden aromatischen Amin in Gegenwart einer wässrigen Mutterlauge, die sich zu mindestens zu 80 Gew.% aus Wasser zusammensetzt, **dadurch gekennzeichnet, dass** Diketen und aromatisches Amin simultan zu im Kreis geführter Mutterlauge in einen Rohrreaktor so dosiert werden, dass die Reaktionstemperatur bei 50 °C bis 100 °C gehalten wird, die Mutterlauge nach der Reaktion bei der genannten Temperatur von 50 °C bis 100 °C abgetrennt und wieder dem Rohrreaktor zugeführt wird, und die erhaltene Acetessigsäurearylamidschmelze mit einer Temperatur von 50 °C bis 100 °C darauf gekühlt und in die industriell anwendbare und handhabbare Form gebracht wird.

## Claims

1. Arylacetoacetamides defined by the general formula 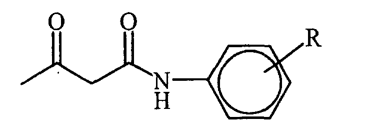 in which R is hydrogen or one or more substituents from the series alkyl, alkoxy or halogen, as a solidified melt in a form which can be used in industry and is easy to handle, which has a water content between 5% by weight and 15% by weight.

2. Arylacetoacetamides according to Claim 1 in the form of flakes, prills, pastilles or tablets.

3. Arylacetoacetamides according to Claim 1 or 2 having a bulk density between 0.3 kg/l and 0.8 kg/l.

4. Acetoacetanilide as arylacetoacetamide according to one of Claims 1 to 3 having a melting point of 50 °C to 70 °C.

5. *o*-Acetoacetanisidide as arylacetoacetamide according to one of Claims 1 to 3 having a water content of up to 12% by weight and a melting point of 50 °C to 80 °C.

6. *o*-Acetoacetotoluidide as arylacetoacetamide according to one of Claims 1 to 3 having a melting point of 80 °C to 100 °C.

7. *m*-Acetoacetoxylidide as arylacetoacetamide according to one of Claims 1 to 3 having a water content of up to 12% by weight and a melting point of 65 °C to 85 °C.

8. *o*-Acetoacetochloroanilide as arylacetoacetamide according to one of Claims 1 to 3 having a water content of up to 12% by weight and a melting point of 75 °C to 95 °C.

9. Arylacetoacetamides according to Claims 1 to 8, obtainable by reacting diketene and the corresponding aromatic amine in the presence of an aqueous mother liquor, at least 80% by weight of which is water, diketene and aromatic amine being metered simultaneously into a tubular reactor to recirculated mother liquor such that the reaction temperature is maintained at 50 °C to 100 °C, the mother liquor is separated off after the reaction at the given temperature of 50 °C to 100 °C and returned to the tubular reactor, and the resulting arylacetoacetamide melt, which has a temperature of 50 °C to 100 °C, is cooled and converted into the form which can be used in industry and is easy to handle.

10. Process for the preparation of arylacetoacetamides by reacting diketene and the corresponding aromatic amine in the presence of an aqueous mother liquor, at least 80% by weight of which is water, **characterized in that** diketene and aromatic amine are metered simultaneously into a tubular reactor to recirculated mother liquor such that the reaction temperature is maintained at 50 °C to 100 °C, the mother liquor is separated off after the reaction at the given temperature of 50 °C to 100 °C and returned to the tubular reactor, and the resulting arylacetoacetamide melt, which has a temperature of 50 °C to 100 °C, is cooled and converted into the form which can be used in industry and is easy to handle.

## Revendications

1. Acétoacétarylamides définis par la formule générale 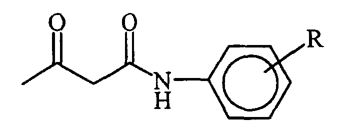 dans laquelle R représente un atome d'hydrogène ou un ou plusieurs substituants choisis dans l'ensemble constitué par des atomes d'halogène et des groupes alkyle et alcoxy, en tant que masse fondue solidifiée sous une forme utilisable et pouvant être manipulée industriellement et ayant une teneur en eau comprise entre 5 % en poids et 15 % en poids.

2. Acétoacétarylamides selon la revendication 1, sous forme d'écailles, de granules, de pastilles ou de comprimés.

3. Acétoacétarylamides selon la revendication 1 ou 2, ayant une densité apparente comprise entre 0,3 kg/l et 0,8 kg/l.

4. Acétoacétanilide en tant qu'acétoacétarylamide selon l'une quelconque des revendications 1 à 3, ayant un point de fusion de 50 °C à 70 °C.

5. Acétoacét-*o*-anisidide en tant qu'acétoacétarylamide selon l'une quelconque des revendications 1 à 3, ayant une teneur en eau de jusqu'à 12 % en poids et un point de fusion de 50 °C à 80 °C.

6. Acétoacét-*o*-toluidide en tant qu'acétoacétarylamide selon l'une quelconque des revendications 1 à 3, ayant un point de fusion de 80 °C à 100 °C.

7. Acétoacét-*m*-xylidide en tant qu'acétoacétarylamide selon l'une quelconque des revendications 1 à 3, ayant une teneur en eau allant jusqu'à 12 % en poids et un point de fusion de 65 °C à 85 °C.

8. Acétoacét-*o*-chloranilide en tant qu'acétoacétarylamide selon l'une quelconque des revendications 1 à 3, ayant une teneur en eau de jusqu'à 12 % en poids et un point de fusion de 75 °C à 95 °C.

9. Acétoacétarylamides selon les revendications 1 à 8, pouvant être obtenus par la réaction de dicétène et de l'amine aromatique correspondante, en présence d'une liqueur-mère aqueuse qui se compose à raison d'au moins 80 % en poids d'eau, le dicétène et l'amine aromatique étant ajoutés de façon réglée à une liqueur-mère mise en circuit dans un réacteur tubulaire, de manière à maintenir la température de la réaction à 50-100°C, la liqueur-mère est séparée après la réaction à la température citée de 50 °C à 100 °C et renvoyée au réacteur tubulaire, et la masse fondue d'acétoacétarylamide obtenue, à une température de 50 °C à 100 °C, est ensuite refroidie et mise sous la forme utilisable et pouvant être manipulée industriellement.

10. Procédé pour la préparation d'acétoacétarylamides par la réaction de dicétène et de l'amine aromatique correspondante, en présence d'une liqueur-mère aqueuse qui se compose à raison d'au moins 80 % en poids d'eau, **caractérisé en ce qu'**on ajoute simultanément le dicétène et l'amine aromatique à la liqueur-mère mise en circuit dans un réacteur tubulaire, de manière à maintenir la température de la réaction à 50-100 °C, la liqueur-mère après la réaction est séparée à la température citée de 50 °C à 100 °C et renvoyée au réacteur tubulaire, et la masse fondue d'acétoacétarylamide obtenue, à une température de 50 °C à 100 °C, est ensuite refroidie et mise sous la forme utilisable et pouvant
